# EUROPEAN PATENT APPLICATION

(11) **EP 3 489 224 A1**
(43) Date of publication of application: **29.05.2019**
(21) Application number: 18213426.2
(22) Date of filing: 14.02.2017
(51) Int. Cl.: C07D 277/40, A61P 13/10, A61K 31/426

(54) **MIRABEGRON PRODRUGS**

(62) Divisional of application: 17156130.1
(71) Applicant: Alfred E. Tiefenbacher (GmbH & Co. KG), 22767 Hamburg (DE)
(72) Inventor: Staver, Ruslan, 22607 Hamburg (DE); Aicher, Daniel, 10823 Berlin (DE)
(74) Representative: Hamm&Wittkopp Patentanwälte PartmbB

(57) **Abstract**

The present invention relates to prodrugs of Mirabegron, their preparation and use in therapy.

## Description

The present invention relates to prodrugs of Mirabegron and their use in therapy.

Mirabegron is a β-3 adrenergic agonist indicated for the treatment of overactive bladder. Extended-release tablets containing 25 mg or 50 mg Mirabegron are commercially available under the tradenames Betmiga® and Myrbetriq®. The use of Mirabegron for the treatment of overactive bladder is described in European patent EP 1 559 427.

Initially, Mirabegron was developed as a remedy for diabetes. European patent EP 1 028 111 discloses the preparation of the dihydrochloride salt of Mirabegron (example 41) and its use for the treatment of diabetes mellitus. According to European patent application EP 1 440 969, the dihydrochloride salt has strong hygroscopicity and is unstable, which poses problems for converting the drug into solid unit dosage forms. As a solution to this problem, the application suggests the crystalline forms α and β of Mirabegron (the free base), which are much less hygroscopic than the dihydrochloride salt.

The free base of Mirabegron can be formulated as a stable matrix-type extended-release tablet containing polyethylene oxide as retarding polymer as well as ferric oxide, as described in European patent EP 1 205 190. However, the commercially available tablets, which are prepared by using the technique described in EP 1 205 190, provide a relatively low bioavailability of the drug, which is even lower if the tablet is taken together with food. Hence, there was a need for improving the oral bioavailability of Mirabegron.

As a solution to the decreased oral bioavailability if Mirabegron is administered together with food, European patent application EP 2 345 410 suggests an extended-release tablet that contains a hydrogel-forming polymer, preferably polyethylene oxide, and an additive that ensures penetration of water into the pharmaceutical composition. Preferably, the additive is selected from polyethylene glycol, polyvinylpyrrolidone, mannitol, sorbitol, sodium chloride, etc. European patent application EP 2 554 168 discloses a multi-layer tablet comprising a Mirabegron-containing layer and release-controlling layers, a tablet in which the drug is contained in a gel formulation composed of gums like locust bean gum and xanthan gum, as well as osmotic pump type tablet formulations. Hence, various tablet formulations have been described in the state of the art in order to minimize the food effect and, thus, enhance the oral bioavailability of Mirabegron.

It was an object of the present invention to provide a pharmaceutical composition containing Mirabegron with improved oral bioavailability. This object is solved by the subject matter as defined in the claims.

It was found that a prodrug of Mirabegron improves the oral bioavailability of the drug. Thus, a prodrug of Mirabegron represented by the following formula (I) is disclosed 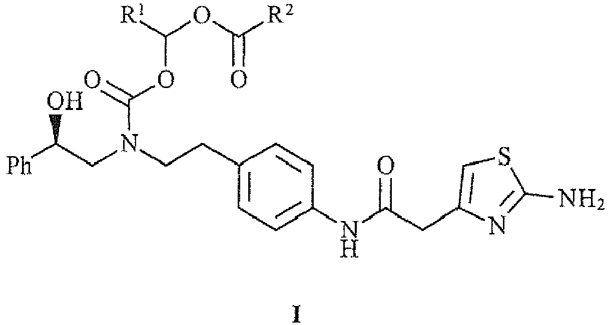 wherein
R¹ represents hydrogen or an optionally substituted, saturated or unsaturated alkyl, and
R² represents an optionally substituted, saturated or unsaturated alkyl, optionally substituted, saturated or unsaturated cycloalkyl, optionally substituted, saturated or unsaturated (cycloalkyl)alkyl, optionally substituted, saturated or unsaturated heterocyclyl, optionally substituted, saturated or unsaturated (heterocyclyl)alkyl, optionally substituted aryl, or optionally substituted heteroaryl,
wherein the substituent of the optionally substituted alkyl, cycloalkyl, (cycloalkyl)alkyl, heterocyclyl, (heterocyclyl)alkyl, aryl and heteroaryl is selected from halogen (F, Cl, Br or I), C₁₋₆-alkoxy, preferably C₁₋₄-alkoxy, more preferably C₁₋₂-alkoxy, phenoxy, (C₁₋₄ alkyl)₂ amino and phenyl
or a pharmaceutically acceptable salt thereof or a solvate thereof.

In case R¹ is not hydrogen, the prodrugs of formula (I) are obtained as a diastereomeric mixture. The diastereomers can be separated by using HPLC or by fractional crystallization.

In the prodrugs, a carbamate is formed with Mirabegron's secondary amine (the N_{phenethyl} atom). A prodrug is an enzymatically reversible derivative of a drug, i.e. a compound that, upon introduction in the appropriate biological system, reverts back to the parent molecule by virtue of enzymatic cleavage. The secondary amine atom of Mirabegron is derivatized as a carbamate, whereby the polar amino group is masked and the drug absorption and, thus, oral bioavailability is enhanced. However, since the carbamate after absorption needs to be hydrolyzed to carbamic acid and the corresponding alcohol, and since the rates of hydrolysis of secondary amines are very slow, an ester moiety is linked to the carbamate moiety in the prodrugs of the present invention in order to initiate the regeneration of the parent amine Mirabegron by an esterase-catalyzed hydrolysis of the ester moiety. The use of a mixed ester-carbamate group as prodrug motif was disclosed in US 5,684,018.

In the prodrugs, usually the saturated alkyl group is C₁₋₇ alkyl, preferably C₁₋₅ alkyl, the unsaturated alkyl group is C₂₋₇ alkenyl, preferably C₂₋₅ alkenyl, the saturated or unsaturated cycloalkyl group is cyclo C₃₋₆ alkyl, the saturated or unsaturated (cycloalkyl)alkyl group is (cyclo-C₃₋₆ alkyl)C₁₋₅ alkyl, the saturated or unsaturated heterocyclyl group is cyclo C₃₋₆ heterocyclyl, in which 1 to 3 carbon atoms of the ring are replaced by O, N or S, the saturated or unsaturated (heterocyclyl)alkyl group is (cyclo C₃₋₆ heterocyclyl)C₁₋₅ alkyl, in which 1 to 3 carbon atoms of the ring are replaced by O, N or S, the aryl group is C₆ or C₁₀ aryl, and the heteroaryl group is C₆ or C₁₀ heteroaryl, in which 1 to 3 carbon atoms of the ring are replaced by O, N or S.

It is more preferred that R¹ represents hydrogen or an unsubstituted, saturated alkyl, and R² represents an unsubstituted, saturated alkyl, or an unsubstituted aryl. For example, R¹ can be selected from hydrogen, methyl, ethyl, n-propyl, iso-propyl, n-butyl, sec-butyl and tert-butyl, and R² can be selected from methyl, ethyl, propyl (n- or iso-), butyl (n-, sec- or tert-), n-pentyl, 2-methylbutyl, 3-methylbutyl and phenyl. Most preferably, R¹ is hydrogen or methyl, and R² is selected from methyl, ethyl, n-propyl, iso-propyl, n-butyl, sec-butyl, n-pentyl and phenyl. Especially preferred is a prodrug in which R¹ is methyl and R² is iso-propyl (Enacarbil-N_{phenethyl}-Mirabegron). Also especially preferred are prodrugs in which R¹ is hydrogen and R² is n-pentyl (hexanoyloxy(methoxy)carbonyl-N_{phenethyl}-Mirabegron) or phenyl (benzoyloxy(methoxy)carbonyl-N_{phenethyl}-mirabegron).

The prodrugs of formula (I) may be prepared as outlined in the following scheme:

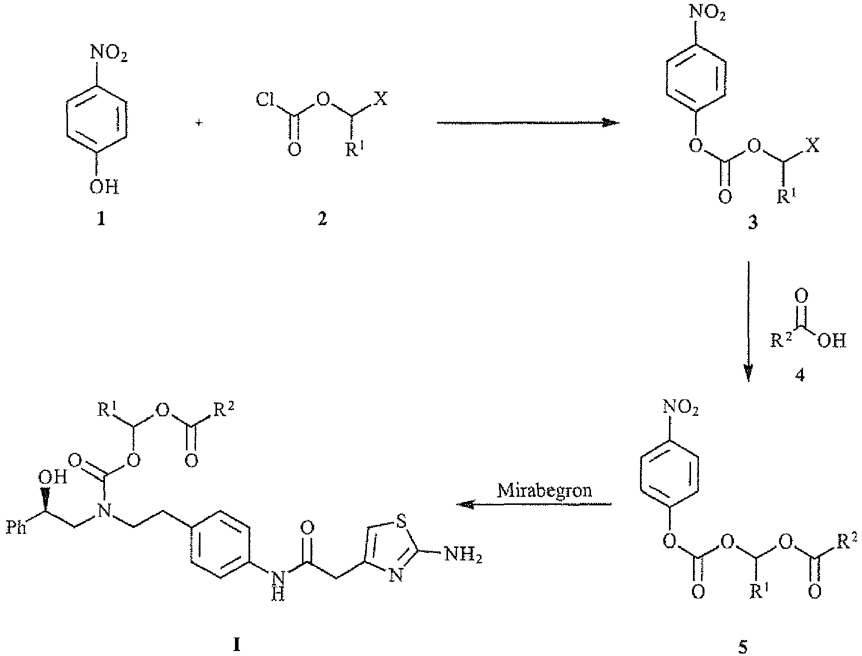

The key intermediate of the process is the mixed carbonate 5 with a para-nitrophenyl moiety that reacts with Mirabegron to yield the N_{phenethyl}-Mirabegron derivatized prodrug I. Hence, a process for preparing a prodrug of formula (I) is disclosed comprising the step of reacting Mirabegron and a carbonate compound represented by the following formula (5) 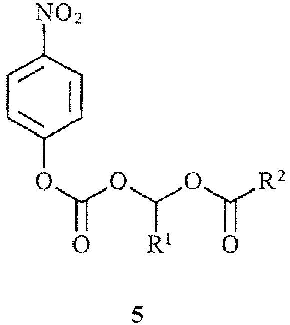 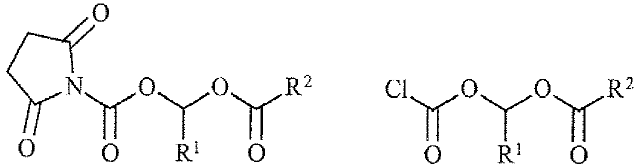 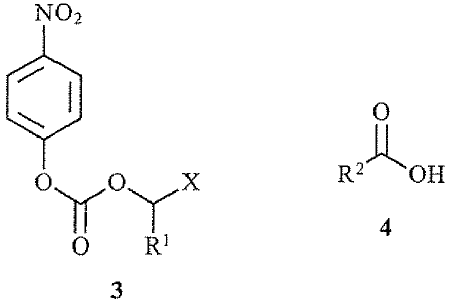 wherein R¹ and R² are as defined above, and X is a leaving group, e.g. Cl, Br, I, alkylsulfonyloxy or arylsulfonyloxy (e.g. methanesulfonyloxy or para-toluene-sulfonyloxy).

The carbonate compound 3 may be prepared by reacting para-nitrophenol and a compound of the following formula 2 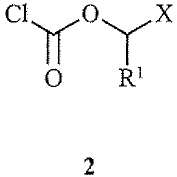 wherein R¹ and X are as defined above.

The present invention further relates to a pharmaceutical composition for oral administration comprising a prodrug of formula (II) shown hereinafter. Preferably, the pharmaceutical composition for oral administration is an extended-release tablet containing the prodrug evenly dispersed within a retarding polymer. Preferably, the extended-release matrix contains polyethylene oxide and/or hydroxypropylmethyl cellulose as a retarding agent as well as polyethylene glycol. The pharmaceutical composition is suitable for the treatment of overactive bladder. The prodrug of formula (II) may be used for the treatment of overactive bladder.

The chemical stability of the prodrugs of formula (I) was tested in simulated gastric fluid (pH 2), fasted-state simulated intestinal fluid (pH 6.5), fed-state simulated intestinal fluid (pH 5.0) and in phosphate buffer (pH 7.5). No hydrolytical cleavage of the prodrug was observed within 48 hours under all conditions.

Compared to the parent compound Mirabegron, it was found that the Tmax is earlier and the Cₘₐₓ is higher after the oral administration of Enacarbil-N_{phenethyl}-Mirabegron to Sprague Dawley rats. The half-life of this prodrug in rat blood plasma was less than 0.5 h and in human blood plasma less than 2.5 h. Almost 100 % esterase cleavage was found in rat blood plasma after 1 h, whereas about 70 % of the prodrug was cleaved within 4 hours in human blood plasma.

The half-life of Benzoyloxy(methoxy)carbonyl-N_{phenethyl}-Mirabegron in rat blood plasma and in human blood plasma was less than 0.5 h. Almost 100 % esterase cleavage was found in rat blood plasma after 1 h, whereas almost 100 % of the prodrug was cleaved within 4 hours in human blood plasma.

Hence, it could be demonstrated that the prodrugs have excellent chemical stability, i.e. they are not hydrolyzed during the passage through the gastrointestinal tract, that they are absorbed faster than the parent compound Mirabegron and that the parent compound is readily set free by esterases after penetration through the biological barrier.

The prodrug of the present invention is represented by the following formula (II) 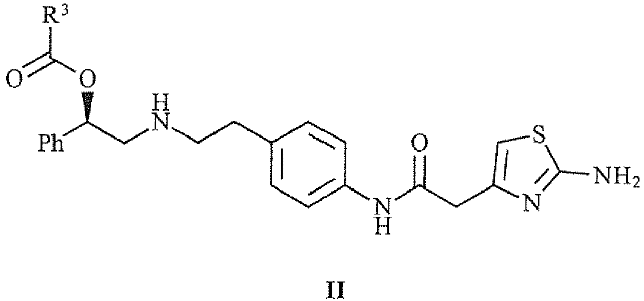 wherein R³ represents an optionally substituted, saturated or unsaturated alkyl, optionally substituted, saturated or unsaturated cycloalkyl, optionally substituted, saturated or unsaturated (cycloalkyl)alkyl, optionally substituted, saturated or unsaturated heterocyclyl, optionally substituted, saturated or unsaturated (heterocyclyl)alkyl, optionally substituted aryl, or optionally substituted heteroaryl,
or a pharmaceutically acceptable salt thereof or a solvate thereof.

In the prodrugs of formula (II), usually the saturated alkyl group is C₁₋₇ alkyl, preferably C₁₋₅ alkyl, the unsaturated alkyl group is C₂₋₇ alkenyl, preferably C₂₋₅ alkenyl, the saturated or unsaturated cycloalkyl group is cyclo-C₃₋₆ alkyl, the saturated or unsaturated (cycloalkyl)alkyl group is (cyclo-C₃₋₆ alkyl)C₁₋₅ alkyl, the saturated or unsaturated heterocyclyl group is cyclo-C₃₋₆ heterocyclyl, in which 1 to 3 carbon atoms of the ring are replaced by O, N or S, the saturated or unsaturated (heterocyclyl)alkyl group is (cyclo-C₃₋₆ heterocyclyl)C₁₋₅ alkyl, in which 1 to 3 carbon atoms of the ring are replaced by O, N or S, the aryl group is C₆ or C₁₀ aryl, and the heteroaryl group is C₆ or C₁₀ heteroaryl, in which 1 to 3 carbon atoms of the ring are replaced by O, N or S. Preferably, R³ represents an unsubstituted, saturated alkyl, an unsubstituted cycloalkyl or an unsubstituted aryl; more preferably, R³ is selected from methyl, ethyl, n-propyl, iso-propyl, n-butyl, sec-butyl, tert-butyl, cyclopropyl, cyclopentyl, cyclohexyl and phenyl.

The prodrug of formula (II) may be prepared by protecting the nitrogen atoms of Mirabegron, e.g. as tris(tert-butyl carbamate), and subsequent reaction of the nitrogen-protected Mirabegron derivative with the corresponding activated acid derivative (e.g., with the respective acyl chloride) followed by deprotection. The invention is further illustrated by reference to the following examples.

### Examples

### Reference Example 1. Enacarbil-N_{phenethyl}-Mirabegron

### Step a: 1-Chloroethyl-p-nitrophenyl carbonate

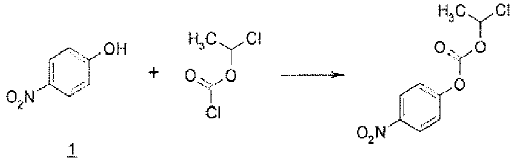

To an ice-cold reaction mixture containing p-nitrophenol (1.39 g, 10 mmol) and triethylamine (10 mmol) in dichloromethane (50 mL) was added 1-chloroethyl chloroformate (1.2 mL, 11 mmol) in 10 ml of dichloromethane. The mixture was stirred at 0°C for 30 min and then at room temperature for 1 hour. The reaction mixture was diluted with water; the organic layer was washed 5-7 times with saturated sodium bicarbonate solution (until the color of the aqueous layer is not very strong yellow-colored) and dried. After removing the solvent under reduced pressure, the residue was dissolved in ethyl acetate, washed 3x with saturated sodium bicarbonate solution, 2x with 10% citric acid, and 2-3x with brine. The organic layer was dried over anhydrous sodium sulfate and evaporated under reduced pressure to give 2.4 g (97%) of the title compound as an off-white solid.

### Yield:

12 g (M 245.6), 48.9 mmol

### Step b: Isobutoxyethyl-p-nitrophenyl carbonate

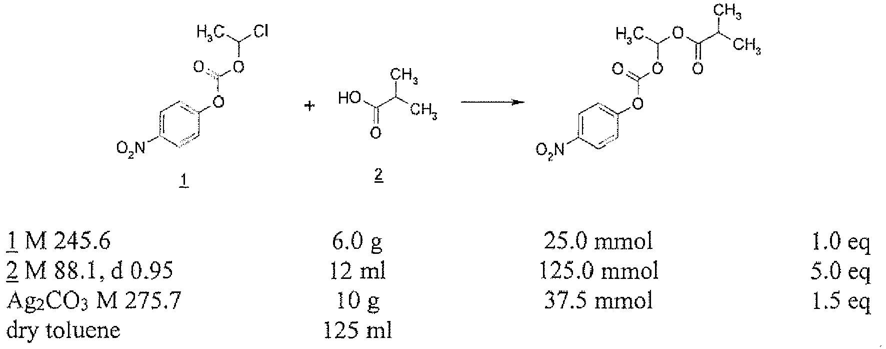

To the solution of 1-chloroethyl-p-nitrophenyl carbonate and isobutyric acid in toluene, silver carbonate is added. The mixture is stirred under reflux overnight in the dark (aluminum foil is used to cover the equipment). The reaction mixture was allowed to cool to room temperature and filtered through a pad of Celite. The filtrate was washed 5x with saturated sodium bicarbonate solution. After removing the solvent under reduced pressure, the residue was dissolved in ethyl acetate, washed 3x with saturated sodium bicarbonate solution and 1-2x with brine. The organic layer was dried over anhydrous sodium sulfate and evaporated under reduced pressure. The isolated substance contains only 20% of the title compound and 80% of an unknown impurity, probably an isobutyric ester.

### Yield:

5 g (purity 20%)

### Step c: Enacarbil-N_{phenethyl}-Mirabegron

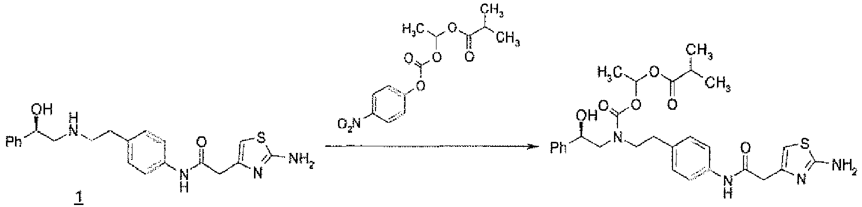

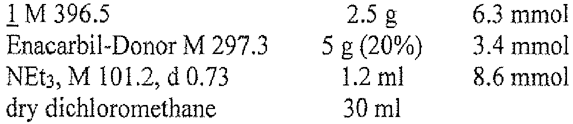

Mirabegron was dissolved in dry dichloromethane. Then triethylamine and the crude Enacarbil-Donor from step b) were added. After stirring for 7 days the reaction mixture was quenched with 100 ml water and a small amount of sodium hydrogen carbonate solution was added. The aqueous layer was extracted twice with dichloromethane. The combined organic layers were evaporated in vacuum. The first purification was achieved by preparative HPLC: Phenomenex Luna Phenyl-Hexyl: MeCN/H₂O/TFA 70:30:0.1. The product fractions were combined and acetonitrile was evaporated in vacuum. The aqueous layer was carefully basified to pH 6 with saturated sodium hydrogen carbonate solution and then brine was added and the aqueous layer was extracted several times with ethylacetate. Further purification was achieved by preparative HPLC: Phenomenex Luna Phenyl-Hexyl: MeCN/H₂O/TFA 50:50:0.1. The product fractions were combined and acetonitrile was evaporated in vacuum. The aqueous layer was carefully basified to pH 6 with saturated sodium hydrogen carbonate solution and then brine was added and the aqueous layer was extracted several times with ethylacetate. The residue was treated with diethyl ether and dried in vacuum and a white solid was obtained.

### Yield:

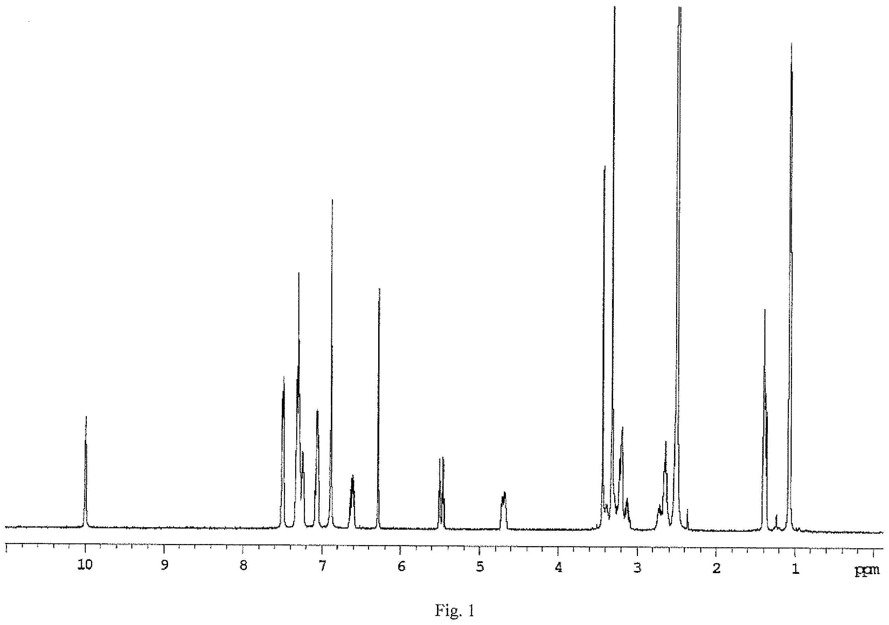

0.3 g
Melting point (m.p.): 58-62°C. MS (ESI) m/z 555.2299 (M+H)⁺.
¹H-NMR (DMSO-d6): Figure 1 (1 Diastereomer)

### Reference Example 2. Benzoyloxy(methoxy)carbonyl-N_{phenethyl}-Mirabegron

### Step a: 1-Chloromethyl-p-nitrophenyl carbonate

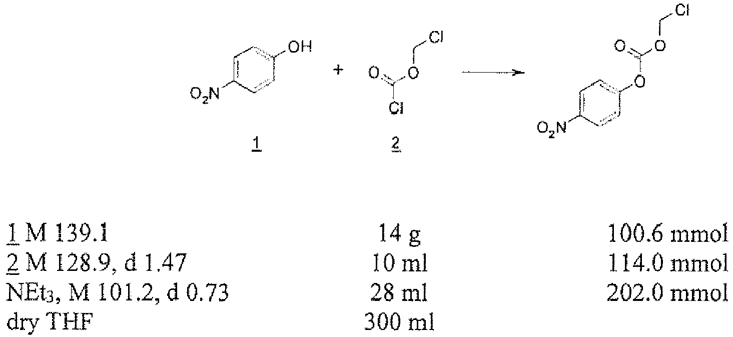

To a -40 °C reaction mixture containing p-nitrophenol and chloromethyl chloroformate in tetrahydrofuran was added triethylamine in one portion. The mixture was stirred at -40°C for 30 min. The solid was filtered off and the filtrate was diluted with ethyl acetate; the organic layer was washed 5 times with a mixture of water, brine and sodium hydrogen carbonate solution. The organic layer was dried over anhydrous sodium sulfate and evaporated under reduced pressure.

### Yield:

20 g (M 231.6), 86.3 mmol, 86%, yellow oil

### Step b: 1-Iodomethyl-p-nitrophenyl carbonate

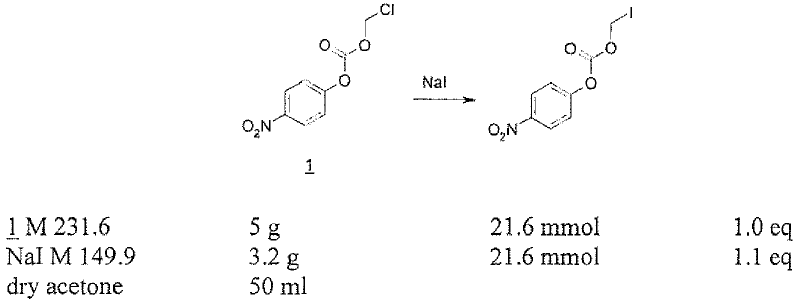

To a solution of 1 in acetone was added NaI in one portion and the mixture was stirred at 50°C overnight. The solvent was evaporated and the residue was dissolved in diethylether. The organic phase was washed 3 times with brine and dried over sodium sulfate.

### Yield:

6 g (M 323.0), 18.6 mmol, 86%

### Step c: Benzoyloxy(methoxy)carbonyl-4-nitrophenol

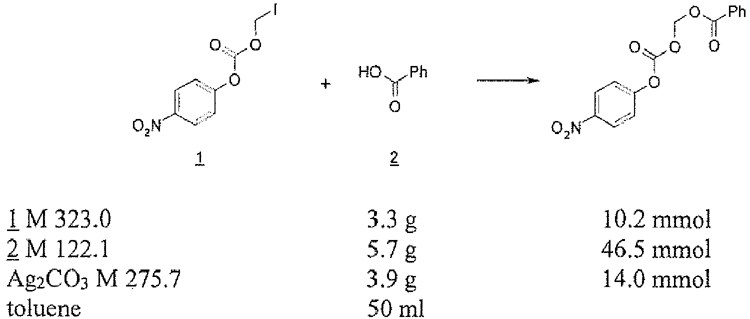

To the solution of 1 and 2 in toluene, silver carbonate is added. The mixture is stirred under reflux 2 h in the dark (aluminum foil is used to cover the equipment). The reaction mixture was allowed to cool to room temperature and filtered. The filtrate was washed with water and brine and dried over anhydrous sodium sulfate. After removing the solvent under reduced pressure, the residue was dissolved in diethylether, washed 3 x with sodium bicarbonate solution and 1 x with brine. The organic layer was dried over anhydrous sodium sulfate and evaporated under reduced pressure.

### Yield:

2.8 g (M 317.3), 8.8 mmol, 86%, yellow oil

### Step d: Benzoyloxy(methoxy)carbonyl-N_{phenethyl}-Mirabegron

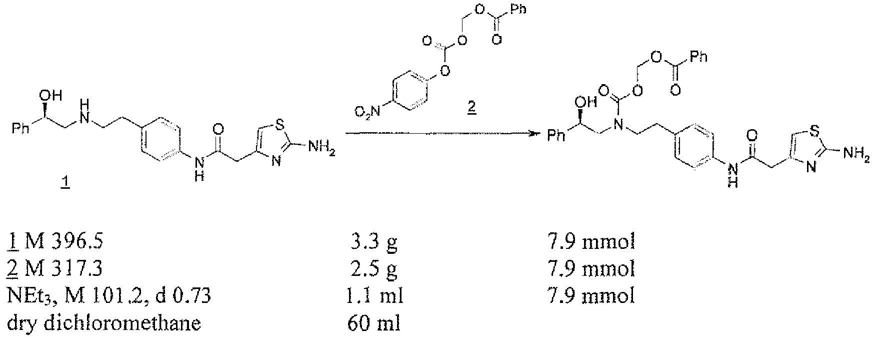

Mirabegron was dissolved in dry dichloromethane. Then triethylamine and benzoyloxy(methoxy)carbonyl-4-nitrophenol were added. After stirring for 2 days the reaction mixture was quenched with 100 ml water and a small amount of sodium hydrogen carbonate solution was added. The aqueous layer was extracted twice with dichloromethane. The combined organic layers were evaporated in vacuum. The first purification was achieved by preparative HPLC: Phenomenex Luna Phenyl-Hexyl: MeCN/H₂O/TFA 60:40:0.1. The product fractions were combined and acetonitrile was evaporated in vacuum. The aqueous layer was carefully basified to pH 6 with saturated sodium hydrogen carbonate solution and then brine was added and the aqueous layer was extracted several times with ethylacetate. Further purification was achieved by repeated preparative HPLC: Phenomenex Luna Phenyl-Hexyl: MeCN/H₂O/TFA 60:40:0.1. The product fractions were combined and acetonitrile was evaporated in vacuum. The aqueous layer was carefully basified to pH 6 with saturated sodium hydrogen carbonate solution and then brine was added and the aqueous layer was extracted several times with ethylacetate. The residue was treated with diethyl ether and dried in vacuum and a light yellow solid was obtained.

### Yield:

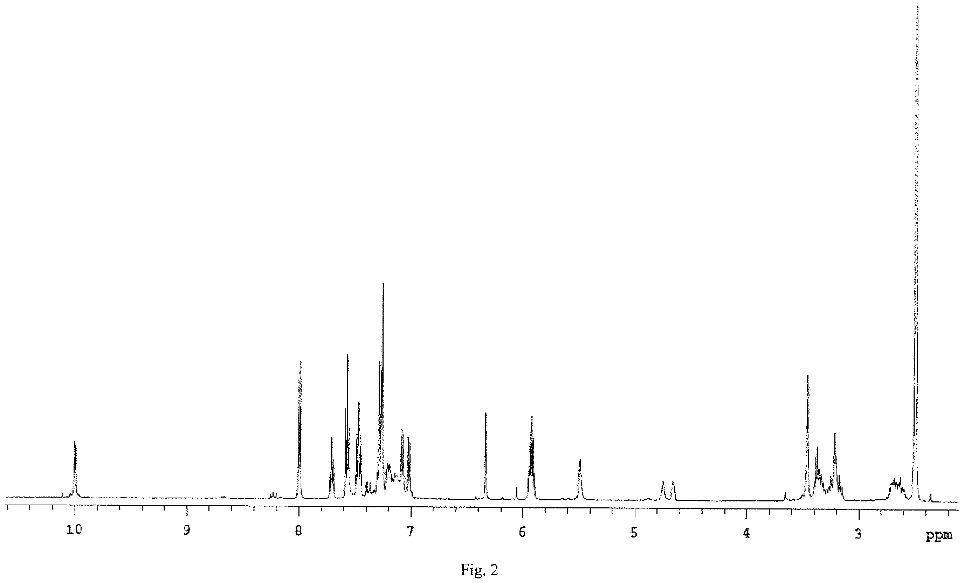

650 mg (M 574.7), 1.1 mmol, 14 %, light yellow solid.
m.p.: 71-74°C. MS (ESI) m/z 575.1961 (M+H)⁺.
¹H-NMR (DMSO-d6): Figure 2

### Reference Example 3. Hexanoyloxy(methoxycarbonyl)-N_{phenethyl}-Mirabegron

Steps a and b correspond to steps a and b of Example 2.

### Step c: Hexanoyloxy(methoxy)carbonyl-4-nitrophenol

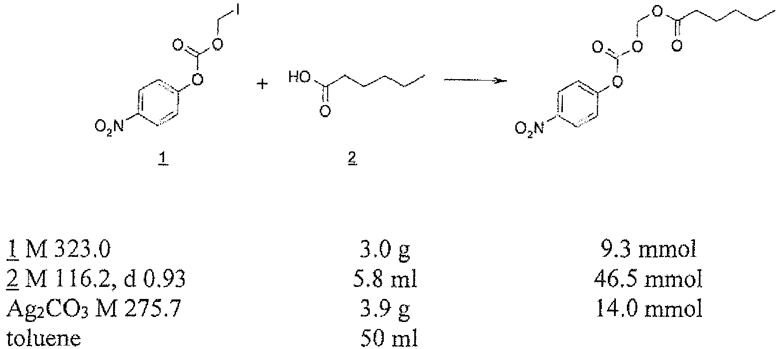

To the solution of 1 and 2 in toluene, silver carbonate is added. The mixture is stirred under reflux 2 h in the dark (aluminum foil is used to cover the equipment). The reaction mixture was allowed to cool to room temperature and filtered. The filtrate was washed with water and brine and dried over anhydrous sodium sulfate. After removing the solvent under reduced pressure, the residue was dissolved in diethylether, washed 3 x with sodium bicarbonate solution and 1 x with brine. The organic layer was dried over anhydrous sodium sulfate and evaporated under reduced pressure.

### Yield:

3.5 g (M 311.3), 8.4 mmol, 90%, yellow oil

### Step d: Hexanoyloxy(methoxycarbonyl)-N_{phenethyl}-Mirabegron

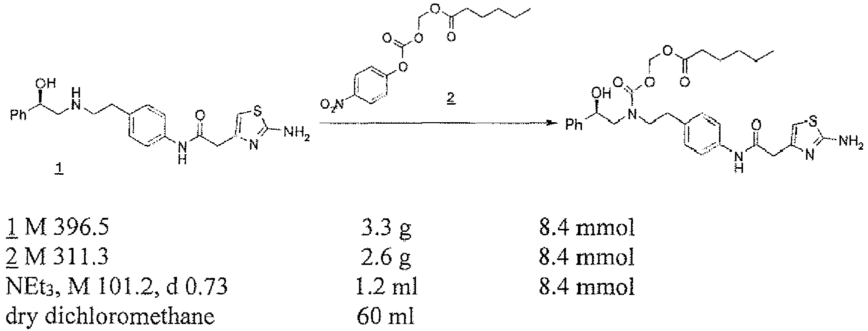

Mirabegron was dissolved in dry dichloromethane. Then triethylamine and hexanoyloxy(methoxy)carbonyl-4-nitrophenol were added. After stirring for 2 days the reaction mixture was quenched with 100 ml water and a small amount of sodium hydrogen carbonate solution was added. The aqueous layer was extracted twice with dichloromethane. The combined organic layers were evaporated in vacuum. The first purification was achieved by preparative HPLC: Phenomenex Luna Phenyl-Hexyl: MeCN/H₂O/TFA 60:40:0.1. The product fractions were combined and acetonitrile was evaporated in vacuum. The aqueous layer was carefully basified to pH 6 with saturated sodium hydrogen carbonate solution and then brine was added and the aqueous layer was extracted several times with ethylacetate. Further purification was achieved by repeated preparative HPLC: Phenomenex Luna Phenyl-Hexyl: MeCN/H₂O/TFA 60:40:0.1. The product fractions were combined and acetonitrile was evaporated in vacuum. The aqueous layer was carefully basified to pH 6 with saturated sodium hydrogen carbonate solution and then brine was added and the aqueous layer was extracted several times with ethylacetate. The residue was treated with diethyl ether and dried in vacuum and a white solid was obtained.

### Yield:

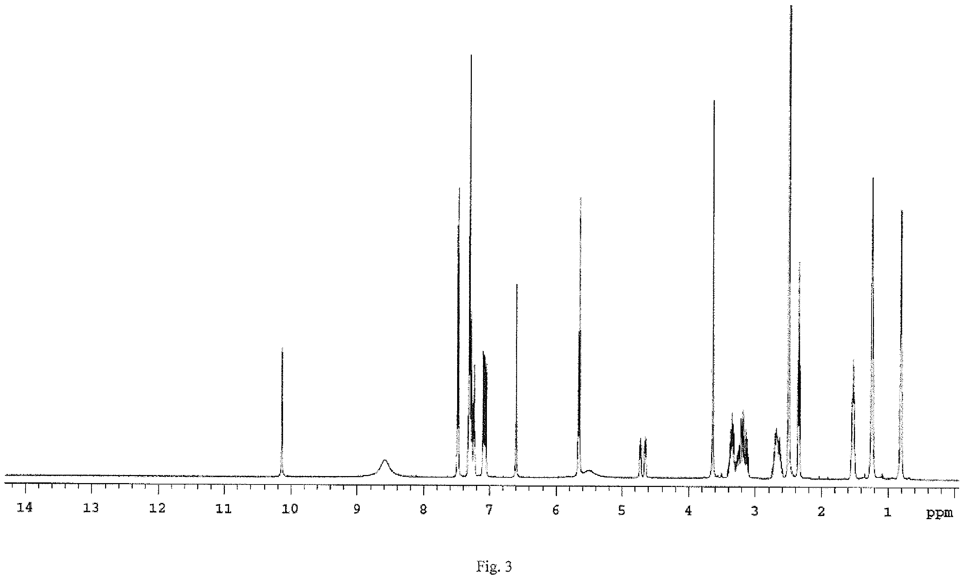

200 mg (M 568.7), 0.35 mmol, 4%, white solid
m.p.: 58-63°C. MS (ESI) m/z 569.2412 (M+H)⁺.
¹H-NMR (DMSO-d6): Figure 3

### Example 4. O-Pivaloyl-Mirabegron

### Step a: N,N',N'-Mirabegron-tris(t-butylcarbamate)

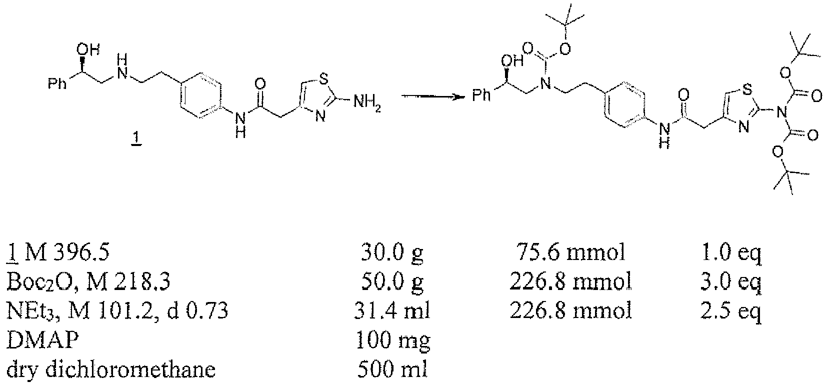

Mirabegron was dissolved in dry dichloromethane. Then triethylamine and Boc₂O were added. DMAP was added and the reaction was stirred overnight. Additional Boc₂O and NEt₃ was added and the mixture was stirred one more night. The reaction mixture was quenched with 500 ml water and brine was added. The aqueous layer was extracted twice with dichloromethane. The combined organic layers were evaporated in vacuum. Purification was achieved by repeated column chromatography: silica gel: chloroform/methanol 97.5:2.5.

### Yield:

17 g (M 696.9), 24.4 mmol

### Step b: O-Pivaloyl-N,N',N'-Mirabegron-tris(t-butylcarbamate)

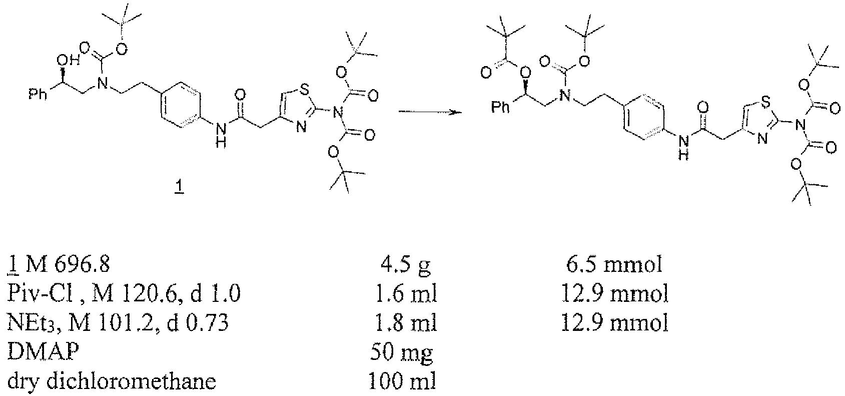

1 was dissolved in dry dichloromethane. Then triethylamine, pivaloyl chloride and DMAP were added and the reaction was stirred overnight. The reaction mixture was quenched with 100 ml water and brine was added. The aqueous layer was extracted twice with dichloromethane. The combined organic layers were evaporated in vacuum. Purification was achieved by preparative HPLC: Phenomenex Luna Phenyl-Hexyl: MeCN/H₂O/TFA 85:15:0. 1. The aqueous layer was carefully basified to pH 6 with saturated sodium hydrogen carbonate solution and acetonitrile was removed in vacuum. Then brine was added and the aqueous layer was extracted several times with ethylacetate. The combined organic layers were dried over sodium sulfate and evaporated in vacuum.

### Yield:

1.6 g (M 781.0), 2.1 mmol

### Step c: O-Pivaloyl-Mirabegron

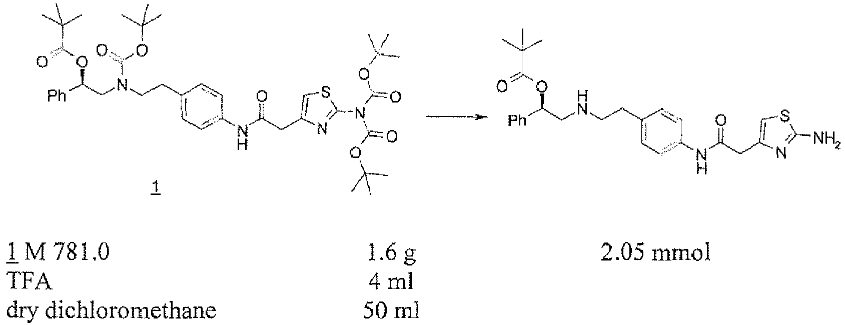

1 was dissolved in dry dichloromethane. Then 2.0 ml TFA were added and the mixture was stirred overnight. 2.0 ml TFA were added and the mixture was stirred one more night. Water was added and the mixture was neutralized with saturated sodium hydrogen carbonate solution. The aqueous layer was extracted twice with dichloromethane and twice with ethyl acetate. The combined organic layers were dried over sodium sulfate and evaporated in vacuum. Further purification was achieved by preparative HPLC: Phenomenex Luna Phenyl-Hexyl: MeCN/H₂O/TFA 40:60:0.1. The aqueous layer was carefully basified to pH 6 with saturated sodium hydrogen carbonate solution and acetonitrile was removed in vacuum. Then brine was added and the aqueous layer was extracted several times with ethylacetate. The combined organic layers were dried over sodium sulfate and evaporated in vacuum. The oily residue was treated with diethyl ether and dried in vacuum and a white foam was obtained.

### Yield:

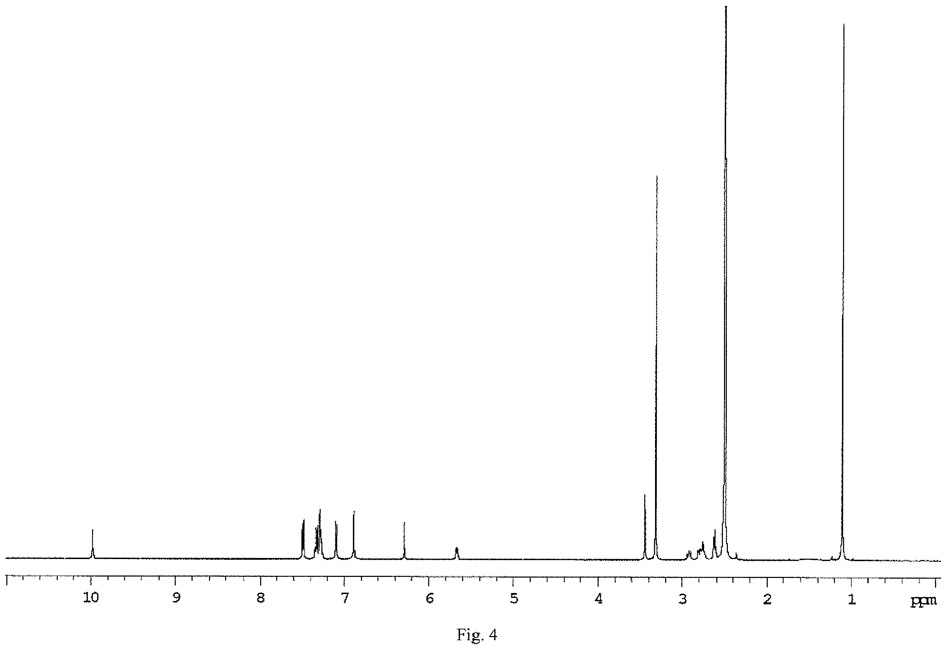

0.5 g (M 480.6), 1.05 mmol
m.p.: 48-50°C. MS (ESI) m/z 481.2227 (M+H)⁺.
¹H-NMR (DMSO-d6): Figure 4

### Example 5. O-Cyclopropanoyl-Mirabegron

Step a corresponds to step a of Example 4.

### Step b: O-Cyclopropanoyl-N,N',N'-Mirabegron-tris(t-butylcarbamate)

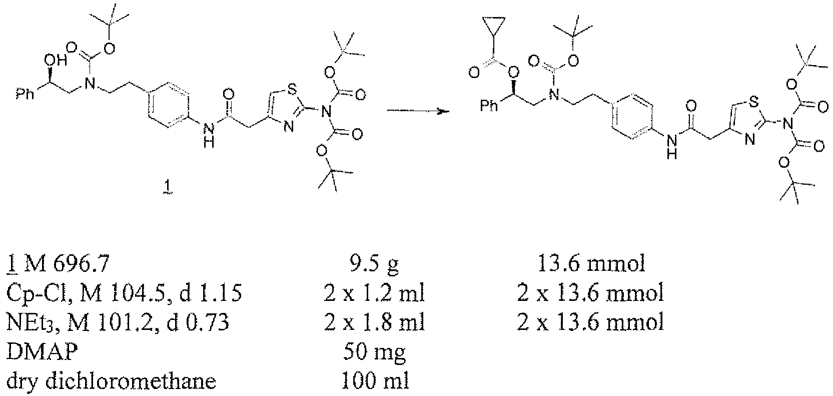

1 was dissolved in dry dichloromethane. Then triethylamine, cyclopropanoyl chloride (Cp-Cl) and DMAP were added and the reaction was stirred overnight. The reaction mixture was quenched with 100 ml water and brine was added. The aqueous layer was extracted twice with dichloromethane. The combined organic layers were evaporated in vacuum. Purification was achieved by repeated preparative HPLC: Phenomenex Luna Phenyl-Hexyl: MeCN/H₂O/TFA 80:20:0.1. The aqueous layer was carefully basified to pH 6 with saturated sodium hydrogen carbonate solution and acetonitrile was removed in vacuum. Then brine was added and the aqueous layer was extracted several times with ethylacetate. The combined organic layers were dried over sodium sulfate and evaporated in vacuum.

### Yield:

3.5 g (M 764.9), 4.6 mmol

### Step c: O-Cyclopropanoyl-Mirabegron

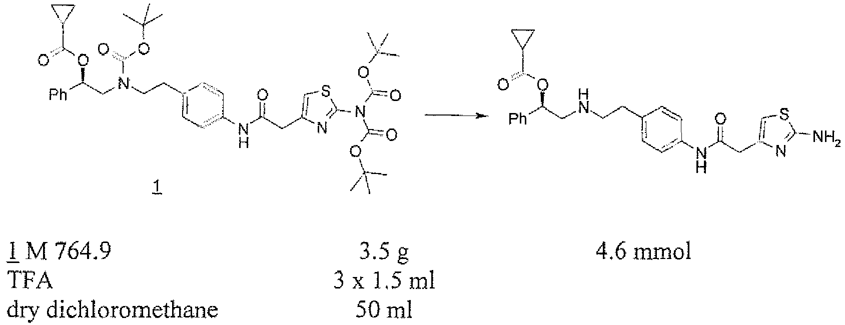

1 was dissolved in dry dichloromethane. Then 1.5 ml TFA were added and the mixture was stirred overnight. Then 1.5 ml TFA were added and once more 1.5 ml TFA and the mixture was stirred one more night. Water was added and the mixture was neutralized with saturated sodium hydrogen carbonate solution. The aqueous layer was extracted twice with dichloromethane and twice with ethyl acetate. The combined organic layers were dried over sodium sulfate and evaporated in vacuum. Further purification was achieved by repeated preparative HPLC: Phenomenex Luna Phenyl-Hexyl: MeCN/H₂O/TFA 30:70:0.1. The aqueous layer was carefully basified to pH 6 with saturated sodium hydrogen carbonate solution and acetonitrile was removed in vacuum. Then brine was added and the aqueous layer was extracted several times with ethyl acetate. The combined organic layers were dried over sodium sulfate and evaporated in vacuum. The residue was treated with diethyl ether and dried in vacuum and a white solid was obtained.

### Yield:

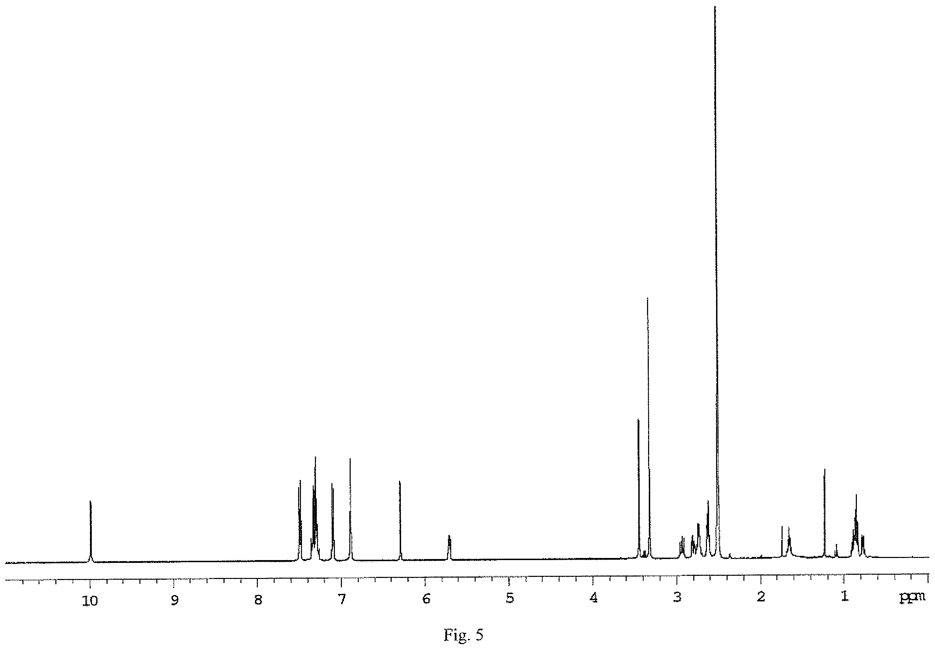

0.2 g (M 464.6), 0.43 mmol
m.p.: 44-47°C. MS (ESI) m/z 465.1917 (M+H)⁺.
¹H-NMR (DMSO-d6): Figure 5

### Example 6. O-Benzoyl-Mirabegron

### Step a: N,N',N'-Mirabegron-tris(t-butylcarbamate)

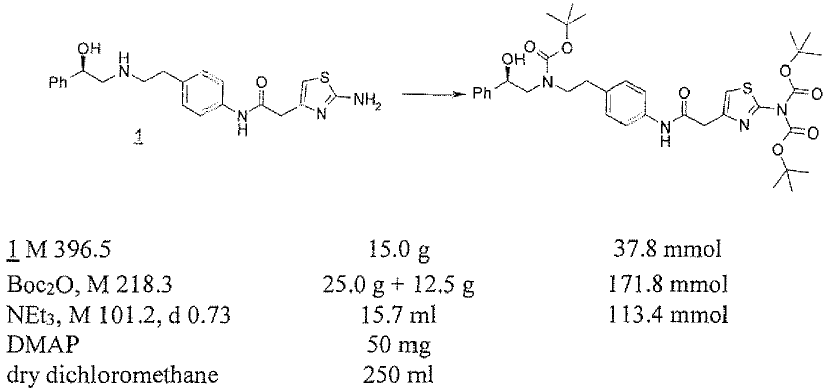

Mirabegron was dissolved in dry dichloromethane. Then triethylamine and Boc₂O were added. DMAP was added and the reaction was stirred overnight. Additional Boc₂O was added and the mixture was stirred one more night. The reaction mixture was quenched with 500 ml water and brine was added. The aqueous layer was extracted twice with dichloromethane. The combined organic layers were evaporated in vacuum. Purification was achieved by repeated column chromatography: silica gel: toluene/ethylacetate 1:1.

### Yield:

13.7 g(M 696.9), 19.7 mmol

### Step b: O-Benzoyl-N,N',N'-Mirabegron-tris(t-butylcarbamate)

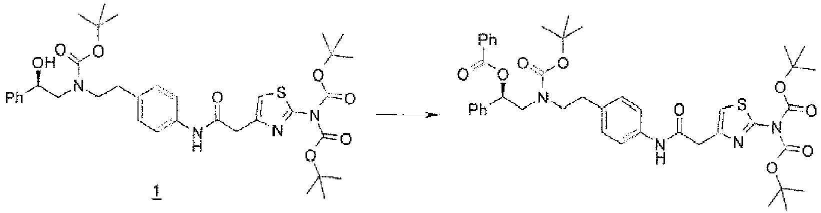

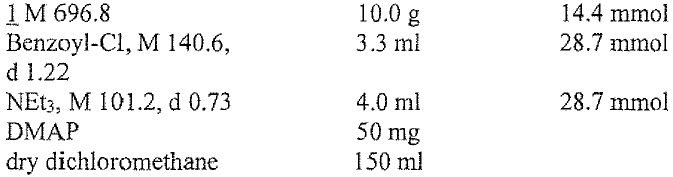

1 was dissolved in dry dichloromethane. Then triethylamine, Benzoyl-Cl and DMAP were added and the reaction was stirred overnight. The reaction mixture was quenched with 100 ml water and brine was added. The aqueous layer was extracted twice with dichloromethane. The combined organic layers were evaporated in vacuum. Purification was achieved by preparative HPLC: Phenomenex Luna Phenyl-Hexyl: MeCN/H₂O/TFA 85:15:0.1. Acetonitrile was removed in vacuum and the aqueous layer was carefully basified to pH 6 with saturated sodium hydrogen carbonate solution. Then brine was added and the aqueous layer was extracted several times with ethylacetate. The combined organic layers were dried over sodium sulfate and evaporated in vacuum.

### Yield:

8.0 g (M 801.0), 10.0 mmol

### Step c: O-Benzoyl-Mirabegron

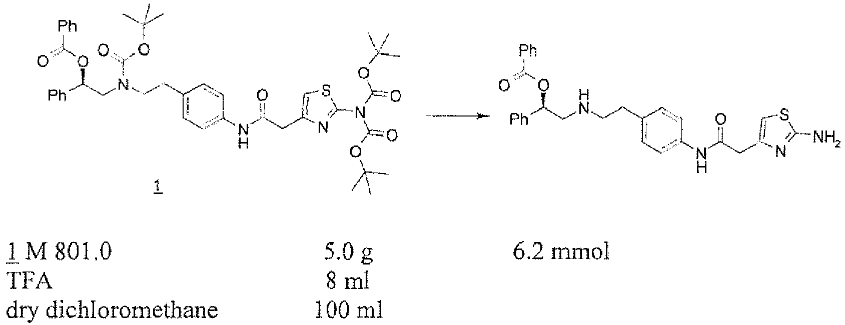

1 was dissolved in dry dichloromethane. Then 4.0 ml TFA were added and the mixture was stirred overnight. 2.0 ml TFA were added and the mixture was stirred one more night. 2.0 ml TFA were added and the mixture was stirred one more night. The solvent and TFA were removed in vacuum. Further purification was achieved by preparative HPLC: Phenomenex Luna Phenyl-Hexyl: MeCN/H₂O/TFA 30:70:0.1. Acetonitrile was removed in vacuum. Then brine and sodium hydrogen carbonate solution was added and the aqueous layer was extracted several times with ethylacetate. The combined organic layers were dried over sodium sulfate and evaporated in vacuum. The residue was treated with diethyl ether and dried in vacuum and a white foam was obtained.

### Yield:

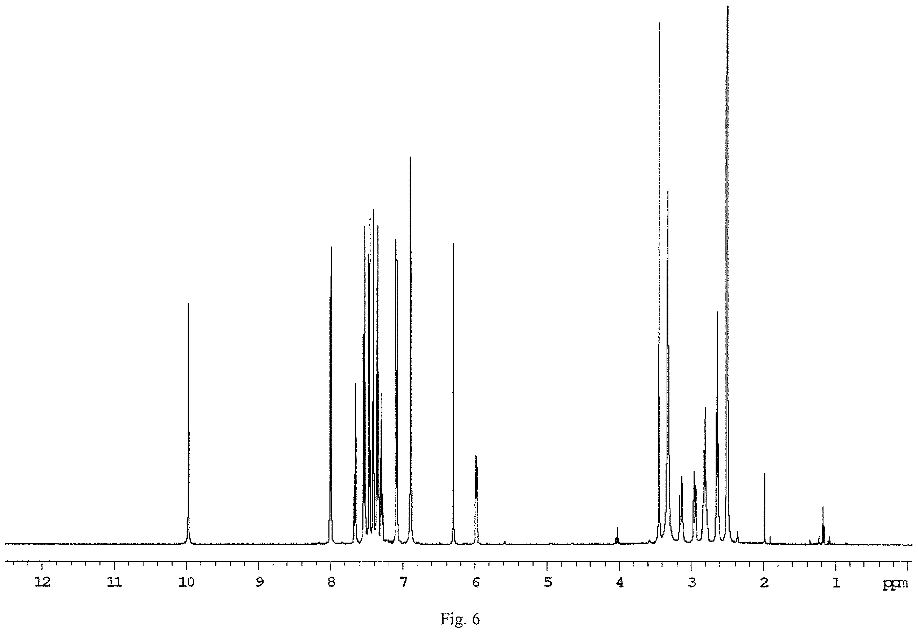

1.1 g (M 500.6), 2.2 mmol
m.p.: 59-64°C. MS (ESI) m/z 501.1962 (M+H)⁺.
¹H-NMR (DMSO-d6): Figure 6

### Example 7. O-Propanoyl-Mirabegron trifluoroacetate

Step a corresponds to step a of Example 6.

### Step b: O-Propanoyl-N,N',N'-Mirabegron-tris(t-butylcarbamate)

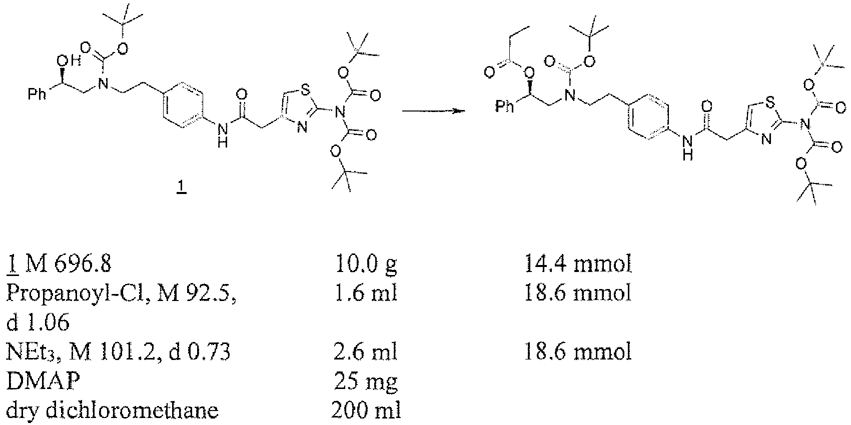

1 was dissolved in dry dichloromethane. Then triethylamine, Propanoyl-CI and DMAP were added and the reaction was stirred overnight. The reaction mixture was quenched with 100 ml water and brine was added. The aqueous layer was extracted twice with dichloromethane. The combined organic layers were evaporated in vacuum. Purification was achieved by preparative HPLC: Phenomenex Luna Phenyl-Hexyl: MeCN/H₂O/TFA 85:15:0.1. Acetonitrile was removed in vacuum and the aqueous layer was carefully basified to pH 6 with saturated sodium hydrogen carbonate solution. Then brine was added and the aqueous layer was extracted several times with ethylacetate. The combined organic layers were dried over sodium sulfate and evaporated in vacuum.

### Yield:

4.2 g (M 752.9), 5.6 mmol

### Step c: O-Propanoyl-Mirabegron trifluoroacetate

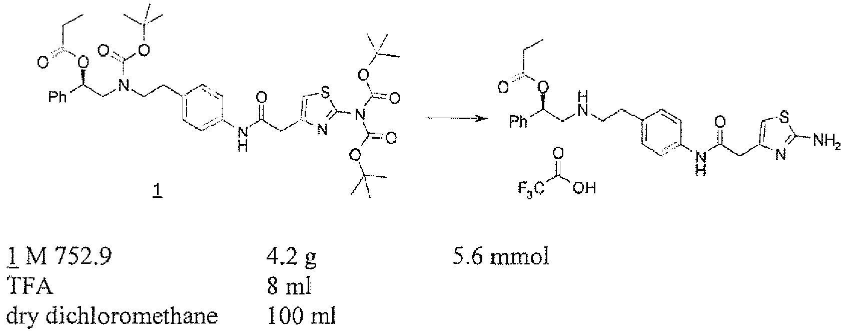

1 was dissolved in dry dichloromethane. Then 4.0 ml TFA were added and the mixture was stirred overnight. 2.0 ml TFA were added and the mixture was stirred one more night. 2.0 ml TFA were added and the mixture was stirred one more night. The solvent and TFA were removed in vacuum. Further purification was achieved by preparative HPLC: Phenomenex Luna Phenyl-Hexyl: MeCN/H₂O/TFA 30:70:0.1 Acetonitrile was removed in vacuum. Then brine and sodium hydrogen carbonate solution was added and the aqueous layer was extracted several times with ethylacetate. The combined organic layers were dried over sodium sulfate and evaporated in vacuum. The residue was treated with diethyl ether and dried in vacuum (decomposition; purity by HPLC is 80%). Purification was achieved once more by preparative HPLC: Phenomenex Luna Phenyl-Hexyl: MeCN/H₂O/TFA 30:70:0.1. Acetonitrile was removed in vacuum. The aqueous residue was lyophilized and white crystals were obtained.

### Yield:

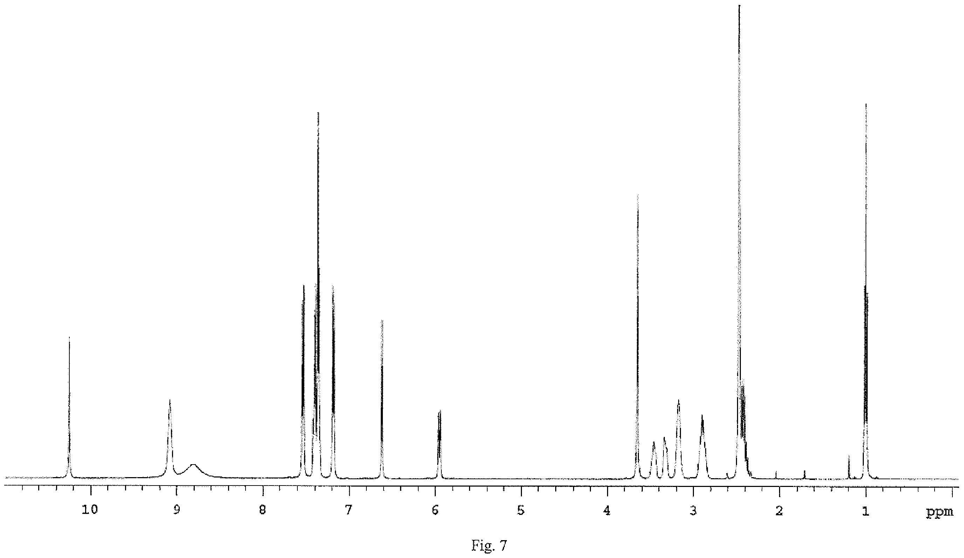

1.0 g (M 566.6)
m.p.: 70-75°C. MS (ESI) m/z 453.1957 (M-CF₃COOH+H)⁺.
¹H-NMR (DMSO-d6): Figure 7

Aspects of the present disclosure are:
1. A prodrug of mirabegron represented by the following formula (I) 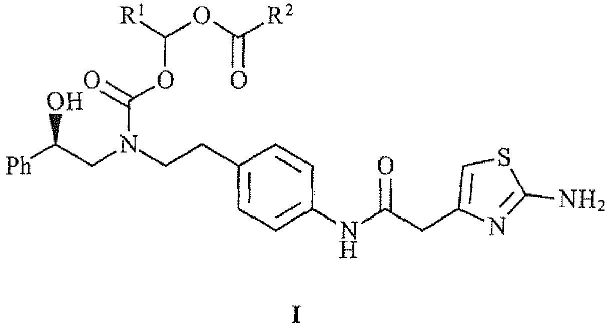 wherein
   - R¹ represents hydrogen or an optionally substituted, saturated or unsaturated alkyl, and
   - R² represents an optionally substituted, saturated or unsaturated alkyl, optionally substituted, saturated or unsaturated cycloalkyl, optionally substituted, saturated or unsaturated (cycloalkyl)alkyl, optionally substituted, saturated or unsaturated heterocyclyl, optionally substituted, saturated or unsaturated (heterocyclyl)alkyl. optionally substituted aryl, or optionally substituted heteroaryl,
   or a pharmaceutically acceptable salt thereof or a solvate thereof.
2. Prodrug according to aspect 1, wherein
   the saturated alkyl group is C₁₋₇ alkyl, preferably C₁₋₅ alkyl, the unsaturated alkyl group is C₂₋₇ alkenyl, preferably C₂₋₅ alkenyl, the saturated or unsaturated cycloalkyl group is cyclo-C₃₋₆ alkyl, the saturated or unsaturated (cycloalkyl)alkyl group is (cyclo-C₃₋₆ alkyl)C₁₋₅ alkyl, the saturated or unsaturated heterocyclyl group is cyclo-C₃₋₆ heterocyclyl, in which 1 to 3 carbon atoms of the ring are replaced by O, N or S, the saturated or unsaturated (heterocyclyl)alkyl group is (cyclo-C₃₋₆ heterocyclyl)C₁₋₅ alkyl, in which 1 to 3 carbon atoms of the ring are replaced by O, N or S, the aryl group is C₆ or C₁₀ aryl, and the heteroaryl group is C₆ or C₁₀ heteroaryl, in which 1 to 3 carbon atoms of the ring are replaced by O, N or S.
3. Prodrug according to aspect 1 or 2, wherein
   - R¹ represents hydrogen or an unsubstituted, saturated alkyl, and
   - R² represents an unsubstituted, saturated alkyl, or an unsubstituted aryl.
4. Prodrug according to aspect 3, wherein
   - R¹ is selected from hydrogen, methyl, ethyl, n-propyl, iso-propyl, n-butyl, sec-butyl and tert-butyl, and
   - R² is selected from methyl, ethyl, propyl (n- or iso-), butyl (n-, sec- or tert-), n-pentyl, 2-methylbutyl, 3-methylbutyl and phenyl.
5. Prodrug according to aspect 4, wherein
   - R¹ is hydrogen or methyl, and
   - R² is selected from methyl, ethyl, n-propyl, iso-propyl, n-butyl, sec-butyl, n-pentyl and phenyl.
6. Prodrug according to aspect 5, wherein
   - R¹ is methyl, and
   - R² is iso-propyl.
7. Prodrug according to aspect 5, wherein
   - R¹ is hydrogen, and
   - R² is n-pentyl or phenyl.
8. A process for preparing a prodrug according to any one of aspects 1-7, wherein the process comprises the step of reacting mirabegron and a carbonate compound represented by the following formula (5) 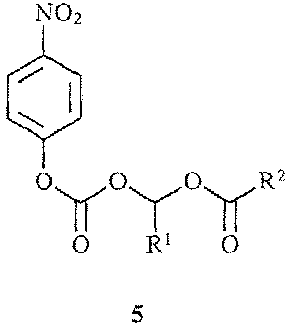 wherein R¹ and R² are as defined above.
9. The process according to aspect 8, wherein the carbonate compound 5 is prepared by reacting a carbonate compound and an acid of the following formulas 3 and 4 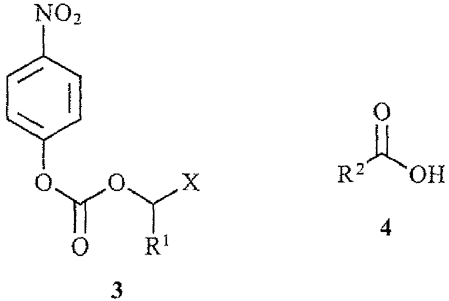 wherein R¹ and R² are as defined above, and X is a leaving group, e.g. CI, Br, I, alkylsulfonyloxy or arylsulfonyloxy.
10. The process according to aspect 9, wherein the carbonate compound 3 is prepared by reacting p-nitrophenol and a compound of the following formula 2 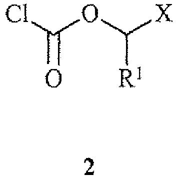 wherein R¹ and X are as defined above.
11. A pharmaceutical composition for oral administration comprising a prodrug according to any one of apects 1-7.
12. A pharmaceutical composition according to aspect 11 for use in a method of treating overactive bladder.
13. A prodrug according to any one of aspect 1-7 for use in a method of treating overactive bladder.

## Claims

1. A prodrug of Mirabegron represented by the following formula (II) 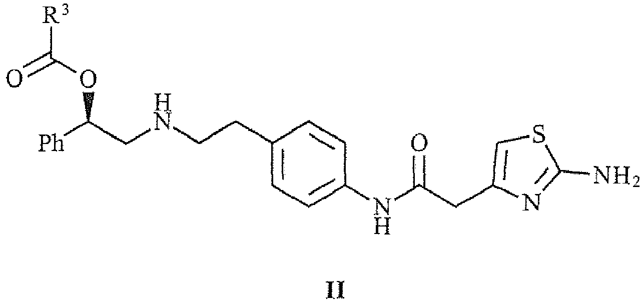 wherein R³ represents an optionally substituted, saturated or unsaturated alkyl, optionally substituted, saturated or unsaturated cycloalkyl, optionally substituted, saturated or unsaturated (cycloalkyl)alkyl, optionally substituted, saturated or unsaturated heterocyclyl, optionally substituted, saturated or unsaturated (heterocyclyl)alkyl, optionally substituted aryl, or optionally substituted heteroaryl,
or a pharmaceutically acceptable salt thereof or a solvate thereof.

2. Prodrug according to claim 1, wherein
the saturated alkyl group is C₁₋₇ alkyl, preferably C₁₋₅ alkyl, the unsaturated alkyl group is C₂₋₇ alkenyl, preferably C₂₋₅ alkenyl, the saturated or unsaturated cycloalkyl group is cyclo-C₃₋₆ alkyl, the saturated or unsaturated (cycloalkyl)alkyl group is (cyclo-C₃₋₆ alkyl)C₁₋₅ alkyl, the saturated or unsaturated heterocyclyl group is cyclo-C₃₋₆ heterocyclyl, in which 1 to 3 carbon atoms of the ring are replaced by O, N or S, the saturated or unsaturated (heterocyclyl)alkyl group is (cyclo-C₃₋₆ heterocyclyl)C₁₋₅ alkyl, in which 1 to 3 carbon atoms of the ring are replaced by O, N or S, the aryl group is C₆ or C₁₀ aryl, and the heteroaryl group is C₆ or C₁₀ heteroaryl, in which 1 to 3 carbon atoms of the ring are replaced by O, N or S.

3. Prodrug according to claim 1 or 2, wherein R³ represents an unsubstituted, saturated alkyl, an unsubstituted cycloalkyl or an unsubstituted aryl.

4. Prodrug according to claim 3, wherein R³ is selected from methyl, ethyl, n-propyl, iso-propyl, n-butyl, sec-butyl, tert-butyl, cyclopropyl, cyclopentyl, cyclohexyl and phenyl.

5. Process for preparing a prodrug according to any one of claims 1-4, in which the amino groups of Mirabegron are protected and the amino-protected Mirabegron is reacted with an activated acid derivative to obtain an amino-protected prodrug of Mirabegron, followed by deprotection to obtain the prodrug of Mirabegron represented by formula (II).

6. A pharmaceutical composition for oral administration comprising a prodrug according to any one of claims 1-4.

7. A pharmaceutical composition according to claim 6 for use in a method of treating overactive bladder.

8. A prodrug according to any one of claims 1-4 for use in a method of treating overactive bladder.
